# EUROPEAN PATENT SPECIFICATION

(11) **EP 3 017 844 B1**
(45) Date of publication and mention of the grant of the patent: **08.09.2021**
(21) Application number: 14819571.2
(22) Date of filing: 26.03.2014
(51) Int. Cl.: A61N 1/39, A61B 5/30, A61B 5/318

(54) **AUTOMATIC EXTERNAL DEFIBRILLATOR, FRONT-END MEASURING SYSTEM AND MEASURING METHOD THEREOF**
AUTOMATISCHER EXTERNER DEFIBRILLATOR, FRONTEND-MESSSYSTEM UND MESSVERFAHREN DAFÜR
DÉFIBRILLATEUR EXTERNE AUTOMATIQUE, SYSTÈME DE MESURE FRONTAL ET SON PROCÉDÉ DE MESURE

(30) Priority: 05.07.2013 CN 201310282001
(43) Date of publication of application: 11.05.2016
(73) Proprietor: Shenzhen Mindray Bio-Medical Electronics Co., Ltd., Shenzhen, Guangdong 518057 (CN); Shenzhen Mindray Scientific Co., Ltd., Shenzhen (CN)
(72) Inventor: WANG, Qi, Shenzhen Guangdong 518057 (CN); CHEN, Dabing, Shenzhen Guangdong 518057 (CN); SHEN, Ning, Shenzhen Guangdong 518057 (CN); LI, Chuanlin, Shenzhen Guangdong 518057 (CN); CEN, Jian, Shenzhen Guangdong 518057 (CN)
(74) Representative: KIPA AB
(86) International application number: PCT/CN2014/074066
(87) International publication number: WO 2015/000312

(56) References cited:
- WO-A1-2012/161940
- WO-A2-2006/016289
- CN-A- 1 921 906
- CN-A- 101 001 668
- CN-A- 101 115 525
- CN-A- 101 119 766
- US-A- 4 919 144
- US-A1- 2006 111 750
- US-A1- 2013 030 307
- US-B1- 6 246 907

## Description

### FIELD OF THE TECHNICAL

The present disclosure relates to a defibrillator applied in medical technology field, and specifically, particularly to an automatic external defibrillator, front-end measuring system and measuring method thereof.

### BACKGROUND

An automatic external defibrillator (AED) is a kind of widely used defibrillation equipment. The AED is accepted by more and more people due to its simple operation, small size, and other advantages. In developed countries such as Europe and America, the AED is widely used in public places such as stations, airports, schools, etc.

Using biphasic wave to defibrillate is a commonly used defibrillating method. Before defibrillating, electrodes are contacted with a proper part of a patient (such as a body) firstly. Then the AED samples and analyses information related to treatment, and determines whether the patient needs electric shock therapy. In detail, firstly, an impedance of a loop of the electrodes and the patient could be measured, and whether the electrodes are well connected with the patient or whether the patient is moving could be detected by measured impedance, which could be a reference factor in adjusting parameters of the AED; secondly, whether there is a pacemaker detected in the patient, and when the pacemaker is detected in the patient a pace pulse with pace marks are synchronous transferred to an analysis algorithm; thirdly, electrocardiograph (ECG) waveform data of the patient is collected through treatment electrodes (2-lead II), and by analyzing the impedance, the pace pulse, and the ECG waveform data to decide whether the patient is ready and whether there is a shockable rhythm. If the shockable rhythm is detected, a charging and discharging device is started to treat the patient. The detection before starting the charging and discharging device is usually named front-end measuring of the defibrillator. It can be seen that whether the patient impedance, the pace pulse, and the ECG waveform data are measured and analyzed accurately is a key of successful defibrillation.

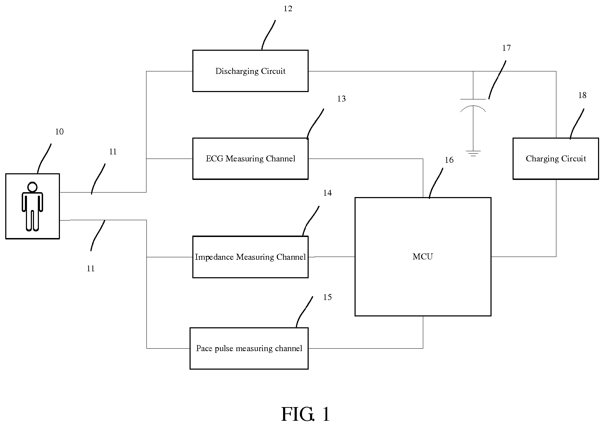

A requirement of a sampling rate and an accuracy of the patient impedance, the pace pulse, and the ECG waveform data are different. Different sampling rates and accuracies require analog-to-digital converters (ADC) with different sampling rates and accuracies. For the ADC, the sampling rate and the accuracy are a couple of conflicting parameters. In other words, the ADC with a high sampling rate has a low accuracy, and the ADC with a high accuracy has a low sampling rate. Thus, in order to meet the sampling rate and accuracy required by different signals, three independent measuring channels are needed to measure the patient impedance, the pace pulse, and the ECG waveform data. Referring to FIG. 1, a front-end measuring system of AED includes three measuring channels, and the three measuring channels are an ECG measuring channel 13, an impedance measuring channel 14, and a pace pulse measuring channel 15. The data measured by the three measuring channels are output to a processor MCU 16 to calculate and analyze. When a shockable rhythm is detected and the impedance value of the patient is within a shockable scope (the electrode is reliably connected to the patient), the processor MCU 16 starts a charging circuit 18 to charge an energy storing device 17. After the charging is finished, an operator is reminded to discharge. A treatment is finished after a human body 10 is discharged through a discharging circuit 12 and electrodes 11.

Currently, a measurement method of the patient impedance, the pace pulse, and the ECG waveform data are described as follows.

The patient impedance could be measured by a carrier wave driving method. In detail, an alternating current (AC) small signal is applied to a human body, and the impedance of the human body is obtained by modulating and analog-digital converting a carrier wave component got on the human body.

The pace pulse could be measured by a hardware comparator. The hardware comparator usually includes a pre-amplificating component, a filter (the filter could be a high-pass filter, a low-pass filter, or a band-pass filter), a post-amplificating component, a double limit comparator, etc.

The ECG waveform data could be measured by an ECG sampling circuit. The ECG sampling circuit could be realized by an alternating-current coupling component and a two-stage amplifying component. Usually, the ECG sampling circuit includes an input-stage defibrillator protecting component, a filtering component, a preamplifying component, a high-pass filtering component, a post-amplifying component, etc.

However, the accuracy of the results obtained by the three independent measuring channels needs to be improved.

US 2006/011175 A1 discloses an automatic external defibrillator (AED) with a discrete sensing pulse for use in configuring a therapeutic biphasic waveform. The sensing pulse is used to determine a patient specific parameter (e.g., thoracic impedance) prior to delivery of the therapy waveform. The defibrillator adjusts the therapy waveform, based on the patient-specific parameter, prior to delivery to the patient.

US 4,919,144 discloses a defibrillator electrocardiogram (ECG) interpreter for indicating whether to treat a patient with a defibrillator. A sequence of digitized samples from a patient's ECG is periodically tested to identify asystole, noise, no-treat and treat conditions. A single result then is stored for each period. The treat result occurs when a patient is experiencing ventricular fibrillation or high rate ventricular tachycardia. The current result and up to two of the results corresponding to immediately previous ECG sample periods are then compared to derive a system output.

### SUMMARY

The invention is defined by the independent claims 1 and 10. In view of this, the present disclosure provides an automatic external defibrillator, a front-end measuring system and a measuring method thereof which could improve accuracy of the measurement.

A front-end measuring system of an automatic external defibrillator, including:
two electrodes;
a carrier wave driving circuit that amplifies a generated alternating current signal and output amplified alternating current signal to the two electrodes;
a sampling circuit that comprises at least one sampling channel, wherein at least one of the sampling channel is a shared sampling channel, the shared sampling channel is a measuring channel shared by at least two different kinds of measured parameters, the measured parameters is selected from a patient impedance, an ECG waveform data, and a pace pulse, the shared sampling channel comprises a difference amplifier and an analogy-to-digital converter, two input ports of the difference amplifier is are coupled to the two electrodes respectively, and an input port of the difference amplifier is coupled to the analog-to-digital converter, the analog-to-digital converter converts an input analog signal to a digital signal by sampling the input analog signal with a predetermined sampling rate and then outputs the digital signal, the predetermined sampling rate of the analogy-to-digital converter is greater than or equal to a maximum sampling rate of the at least two different kinds of measured parameters corresponding to the shared sampling channel; and
a processor, coupled to the sampling circuit, which generates the alternating current signal, outputs the alternating current signal to the carrier wave driving circuit, receives the digital signal output by the sampling circuit, and extracts the digital signal according to a sampling rate and an accuracy required by corresponding measured parameter.

An automatic external defibrillator, including a front-end measuring system described before.

A front-end measuring method of an automatic external defibrillator, including:
providing a carrier wave signal to a patient via two electrodes connected to a proper part the patient;
detecting and sampling signals on the two electrodes , wherein at least two different kinds of measured parameters use one shared sampling channel, the measured parameter is any one of a patient impedance, an ECG waveform data, and a pace pulse, a predetermined sampling rate of the shared sampling channel is greater than or equal to a maximum sampling rate of the at least two different kinds of measured parameters corresponding to the shared sampling channel; and
receiving a digital signal output by the shared sampling channel via a processor, and then extracting the digital signal according to a sampling rate and an accuracy required by corresponding measured parameter.

In the front-end measuring system of an automatic external defibrillator, the automatic external defibrillator and the measuring method, original signals are oversampled by an analog to digital converter, and then the oversampled signals are extracted according to a predetermined ratio to realize that one shared channel could be shared to measure any two or three of the following three parameters: the patient impedance, the ECG waveform date, and the pace pulse of the patient. Therefore, the number of hardwares used in the sampling circuit could be reduced, problems of component dispersity and node interference caused by the large number of the hardwares could be reduced, and the accuracy of measuring result of the instrument could be improved too.

### BRIEF DESCRIPTION OF THE DRAWINGS

In order to illustrate technical solutions according to embodiments of the disclosure, the drawings to be used in the description of the embodiments of the disclosure will be described briefly hereinafter. The drawings described hereinafter include only some embodiments related to the present disclosure. Other drawings may be determined by those skilled in the art based on these drawings without any creative effort.
FIG.1 is a schematic diagram of a defibrillator in current technology.
FIG.2 is a schematic diagram of a front-end measuring system of a defibrillator according to an embodiment.
FIG.3 is an equivalent circuit diagram of electrodes connecting with human body according to an embodiment.
FIG.4 is a schematic diagram of a processor for extracting sampling signal according to an embodiment.
FIG.5 is a detailed equivalent circuit diagram of electrodes connecting with human body according to an embodiment.
FIG.6 is a flowchart diagram of a front-end measuring system of a defibrillator according to an embodiment.
FIG. 7 is a flowchart diagram of recognizing a tested load according to an embodiment.
FIG.8 is a flowchart diagram of recognizing a tested load according to another embodiment.

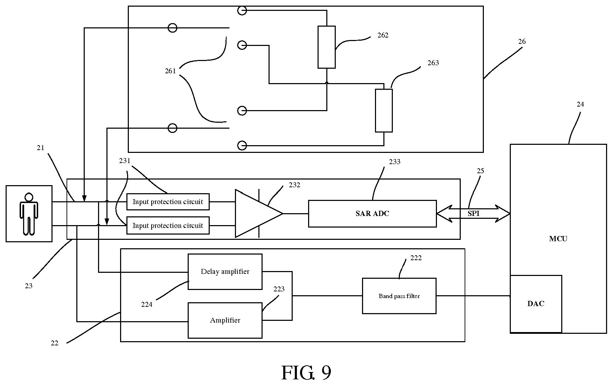

FIG.9 is schematic diagram of a front-end measuring system including calibration circuits according to an embodiment.

### DETAILED DESCRIPTION OF ILLUSTRATED EMBODIMENTS

Further description of present disclosure will be illustrated, which includes embodiments in drawings and detailed embodiments. In the following description, a defibrillator applied on a human body (a patient is a human) is used as an example. It is understood by those skilled in the art that the defibrillator can also be applied on animal patients.

When a patient impedance, ECG waveform data, and pace pulse are measured and analyzed by a front-end measuring system of a defibrillator, the patient impedance, ECG waveform data, and pace pulse are detected from electrodes. However, a requirement of sampling rates and accuracies of the patient impedance, ECG waveform data, and pace pulse are different. When measuring the patient impedance, a small alternating current (AC) signal is applied to the human body. A frequency of the AC signal has a basic requirement that the frequency of the AC signal could not be too small. There is clinical evidence to prove that the AC signal with a higher frequency does less harm to the human body. In consideration of actual system implementation and data processing, the AC signal with a frequency of 32 kHz could be applied to the human body. In order to ensure that the AC signal with the frequency of 32 kHz could be sampled almost without distortion, the sampling rate is at least 8 times of the frequency of the AC signal, that is 256 kSa/s. But the accuracy required by the patient impedance measuring is not so high, usually, 12 to 13 bits are enough. For ECG measuring, the sampling accuracy required by ECG algorithm analysis is 5 uV/lsb, thus, the accuracy required is more than 20 bits. A main focus of an AED treatment system is the component of the ECG signal which frequency is between 0.5-40 HZ. Thus, the sampling rate is 10 times of the frequency of the component, that is 500sa/s. For pace pulse measuring, the width of the pace pulse needed to be identified is 0.1ms∼2ms, and the amplitude of the pace pulse needed to be identified is 2mv∼700mv. The sampling rate is at least 10 ksa/s, in order to ensure that at least one point could be sampled from a pace pulse a width of which is 0.1ms. Pace pulse measuring requires to identify a 2 mV signal, so the sampling accuracy of the pace measuring is at least 200 uV/lsb. In other word, the accuracy is required to reach 16 bits. From the above description, it can be seen that in the patient impedance measuring, ECG signal measuring, and pace channel measuring, the sampling rate required by the patient impedance measuring is the highest and the accuracy required by the patient impedance measuring is the lowest, the sampling rate required by the ECG signal measuring is in the middle and the accuracy required by the ECG signal measuring is the highest, the sampling rate required by the pace pulse measuring is the lowest and the accuracy required by the pace pulse measuring is in the middle .

In one embodiment, one shared channel could be shared to measure any two or three of the following three parameters: the patient impedance, the ECG waveform date, and the pace pulse of the patient. The most important component in the shared channel is an analog-to-digital converter (ADC). Sharing the sampling channel means sharing the ADC. However, for an ADC, only one sampling rate and accuracy are taken to sample an analog signal at the same time, and the sampling rate and the accuracy are a couple of conflicting parameters. Thus, it is difficult for the shard ADC to meet a requirement of the sampling rates and accuracies required by more than two kinds of measured parameters. In the embodiment, the signal is sampled by the ADC according to a maximum sampling rate of the measured parameters. In other words, the signal is oversampled. Then oversampled signal is extracted according to a predetermined rate in subsequent processing. After that extracted signal could meet sampling rate requirement of corresponding measured parameter. At the same time, the accuracy of the oversampled signal is adjusted to meet the accuracy requirement of corresponding measured parameter. In other embodiment, the shared sampling channel could be shared by the patient impedance measuring and the pace pulse measuring, and the other sampling channel could only be used by the ECG waveform data measuring. Or, the shared sampling channel could be shared by the patient impedance measuring and the ECG waveform data measuring, and the other sampling channel could only be used by the pace pulse measuring. Or, the shared sampling channel could be shared by the ECG waveform data measuring and the pace pulse measuring, and the other sampling channel could only be used by the patient impedance measuring. If the greater sampling rate of the two measured parameters is equal to the sampling rate of the ADC in the shared sampling channel, the processing of extracting the oversampled signal corresponding to the measured parameter requiring the greater sampling rate could be omitted.

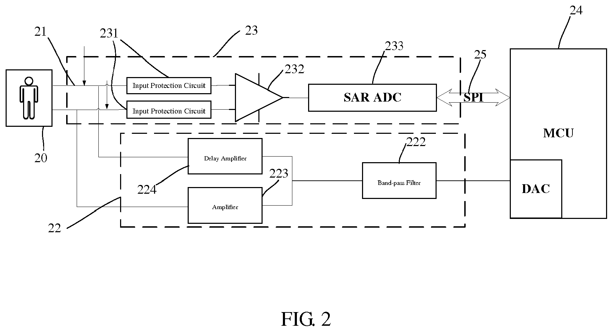

Referring to FIG.2, in an embodiment, a front-end measuring system of an AED includes a sampling channel shared by the patient impedance measuring, the ECG waveform data measuring, and the pace pulse measuring. The front-end measuring system of the AED includes two electrodes 21, a carrier wave driving circuit 22, a sampling circuit 23, and a processor 24. An AC signal output port of the processor 24 is connected to an input port of the carrier wave driving circuit 22. An AC signal is divided into two signals with different phases by the carrier wave driving circuit 22, and then the two signals are sent to the two electrodes 21 through two output ports of the carrier wave circuit 22 respectively. The sampling circuit 23 includes the sampling channel shared by the patient impedance measuring, the ECG waveform data measuring, and the pace pulse measuring. Two input ports of the sampling circuit 23 are connected to the two electrodes 21 respectively. An output port of the sampling circuit 23 is connected to the processor 24 via a data interface 25. The sampling circuit 23 collects original signals representing patient impedance, the ECG waveform data, and the pace pulse.

The electrodes 21 are conductors which could be cylindrical or schistose.

The carrier wave driving circuit 22 includes a band pass filter 222, an amplifier 223, and a delay amplifier 224 in one embodiment. An input port of the band pass filter 222 is coupled to an output port of the processor 24 for outputting the alternating current signal. An output port of the band pass filter 222 is coupled to input ports of the amplifier 223 and the delay amplifier 224 respectively. Output ports of the amplifier 223 and the delay amplifier 224 are coupled to the two electrodes 21 respectively. A phase of the AC signal output by the delay amplifier 224 is a predetermined phase delayed compared to a phase of the AC signal output by the amplifier 223. In detail, the delay amplifier 224 can be an inverting amplifier. A phase of the AC signal output by the inverting amplifier is contrary to the phase of the AC signal output by the amplifier. When two signals which phases are contrary to each other are applied to the human body 20, effects of the two signals can be offset each other.

The sampling circuit 23 includes a difference amplifier 232 and an analog-to-digital converter 233 in one embodiment. Two input ports of the difference amplifier 232 are respectively coupled to the two electrodes 21, and an output port of the difference amplifier 232 is coupled to the analog-to-digital converter 233. The difference amplifier 232 converts a differential analog signal of the human body to a signal-end analog signal, and outputs the signal-end analog signal. It is in favor of restraining common-mode signal of the human body. If the magnification of the difference amplifier 232 is controlled in a range of 2-4, a polarization voltage range provided could be +/-1V. In order to improve a common-mode rejection ratio and a polarization voltage range of the difference amplifier 232, a providing voltage of the difference amplifier 232 could be further improved. The analog-to-digital converter 233 converts an analog signal to a digital signal by sampling the analog signal with a predetermined rate, and outputs the digital signal to the processor 24. The analog-to-digital converter 233 of the sampling circuit 23 could be a successive approximation register analog-to-digital converter (SAR ADC). The analog-to-digital converter 233 is set to oversample, wherein, the sampling rate is greater than or equal to a maximum sampling rate of the patient impedance, ECG waveform data, and pace pulse. Since the sampling rate required by the patient impedance measuring is the maximum, the sampling rate of the analog-to-digital converter 233 set is greater than or equal to the sampling rate required by the patient impedance measuring. In order to simplify subsequent processing of the processor 24, the sampling rate of the analog-to-digital converter 233 is set to equal to the sampling rate required by the patient impedance measuring, for example 256 kSa/s. The processor 24 controls a sampling sequence of the analog-to-digital converter 233 via a data interface 25. For example, the processor 24 provides a clock signal to control the sampling rate of the analog-to-digital converter 233.

The sampling circuit 23 also includes two input protection circuits 231 in one embodiment. The two input protection circuits 231 are connected between two input ports of the difference amplifier 232 in the sampling channel and corresponding electrodes respectively. The input protection circuit 231 absorbs high voltage energy produced by the defibrillator, and avoids damage to low-voltage components of the sampling circuit when defibrillating. In specific realization, the input protection circuit could be a gas-discharge tube which is connected to the ground. The gas-discharge tube conducts AC high voltage to the ground when defibrillating. In specific realization, the input protection circuit also limits the input voltage to a predetermined electric potential in an insertion way by using a high energy pulse resistance and a diode.

The workflow of the front-end measuring system of the AED described above is described in detail as follows.

The processor 24 generates an AC signal with a certain characteristic via an analog-to-digital converter which is build in the processor or in external of the processor. The AC signal could be a sinusoidal wave or a square wave. It is preferable that the AC signal is a sinusoidal wave, because the sinusoidal wave could avoid the carrier wave signal causing an aliasing and interference to the ECG signal.

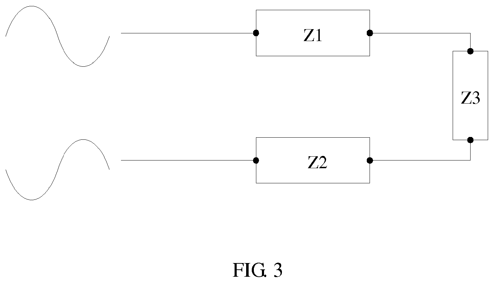

The AC signal is provided to the human body after filtered by the band pass filter 222 and amplified by the amplifier. When the electrodes 21 is connected with the human body, a connecting impedance exists between the electrodes 21 and the human body science the human body is equal to an impedance , and an equivalent circuit diagram is shown in FIG. 3. In FIG. 3, Z1 and Z2 are equivalent output impedances of the carrier driving circuit. The equivalent output impedances are connecting impedances between the electrodes and the human body. Z3 is measured impedance, and the measured impedance is an impedance of the human body. Partial voltage signals are generated on two terminals of the electrodes 21 when the AC signal is provided to the human body via the electrodes.

The sampling circuit 23 adjusts and collects the partial voltage signals generated by the AC signal being applied to the human body. In detail, an original signal of the electrodes is detected by direct current coupling method. In order to get a higher resolution and sampling accuracy, the original data is oversampled by the analogy-to-digital converter with higher sampling rate, and then the oversampled signal is extracted. The sampling accuracy of the analogy-to-digital converter is usually 12 bit∼13 bit, and the sampling rate is more than 500 kSa/s. Usually a carrier frequency of the AC signal is in a range of 20 kHz∼30 kHz, so a bandwidth of the sampling circuit 23 set is about 40kHz to ensure that the impedance signal could be sampled. The sampling rate of the analog-to-digital converter is set to be 500 kHz, thus the carrier wave signal could be completely digitalized.

The processor 24 receives a digital signal output by the sampling circuit 23, and respectively extracts the digital signal by three channels according to a sampling rate and an accuracy required by the impedance measurement, the ECG waveform data measuring, and the pace pulse measuring. The processor 24 includes a patient impedance calculating and processing module, an ECG wave data extracting and processing module, and a pace pulse processing module. An impedance detecting value, an ECG detecting original data, and the pace detecting original signal are respectively obtained after the digital signal is respectively processed by the patient impedance calculating and processing module, the ECG wave data extracting and processing module, and the pace pulse processing module.

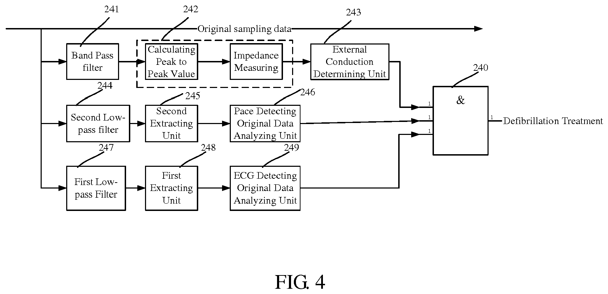

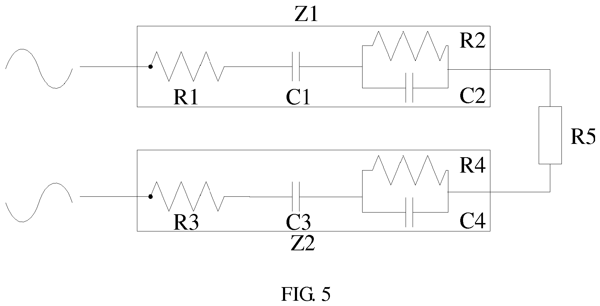

Referring to FIG. 4, for the impedance calculating and processing module, the sampling rate of the front-end meets the sampling rate required by the measurement of the patient impedance, therefore, it is not necessary to do an extracting processing to the digital signal. The patient impedance calculating and processing module includes a band pass filter 241, and a calculating unit 242. The band pass filter 241processes the digital signal output by the shared sampling channel. The calculating unit 242 calculates the patient impedance based on the filtered digital signal. The calculating unit 242 also outputs value of the patient impedance to an external conduction determining unit 243. The external conduction determining unit 243 determines whether the electrodes contact well with the patient. The patient impedance calculating and processing module firstly band-pass filters the digital signal to eliminate noise interference. The central frequency of the filter bandwidth could be 30 KHZ. The patient impedance calculating and processing module calculates the value of the patient impedance based on the filtered digital signal. For example, the value of the patient impedance is obtained by calculating a peak to peak value of waveform formed by filtered digital signal. Next, the patient impedance calculating and processing module analyzes the value of the patient impedance, for example, determines whether the electrodes are connect with the human body well by comparing the value of the patient impedance calculated with a predetermined range. In one embodiment, a method of determining whether the electrodes connected well with the patient is described in detail as follows. The processor 24 outputs two AC signals with different frequencies, and determines whether the two electrodes 21 are well connected to a patient according to a signal collected by the sampling circuit. The equivalent circuit diagram of the electrodes 21 connecting with the patient could be referred to FIG. 5. Z1(Z2) includes R1(R3), C1(C3), R2//C2(R4//C4) connected in series. R1(R3) is a defibrillation pulse energy absorbing resistance. C1(C3) is an AC coupling capacitance, and is configured to avoid direct current being coupled to the human body to cause damage to the human body. R2//C2(R4//C4) is a contact impedance between the electrodes and the human body. R5 is patient thoracic impedance. In the actual clinical environment, R2//C2(R4//C4) could be affect by bad contact between the electrodes and the human body (such as dry skin, road bumps, and so on), and lead to a greater impedance detecting value or a delay of defibrillation treatment caused by electrodes falling , and lead to a rescue failure. R2//C2, R4//C4 could change with different connecting degree, the connecting degree is good, or poor, etc. When the carrier wave frequency of the AC signal exceeds a predetermined value, an influence of a RC mesh consisting of R2//C2 and R4//C4 to impedance of the human body could almost be omitted. When the electrodes are well connect with the human body, a value of R2//C2 (R4//C4) mesh is small and basicly fixed, a high pass cutoff frequency is higher, and the impedances driven by two carrier wave with different frequencies are almost the same. When the electrodes are poorly connected with the human body or the human body is moving, the value of R2//C2 (R4//C4) mesh is larger or oscillating, corresponding high pass cutoff frequency is low, and the impedances driven by two carrier wave with different frequencies are very different. From description above, it can be seen that a connecting condition of the external electrodes could be obtained by comparing two measured values of impedance driven by two carrier waves with different frequencies, and a warning message could be provided.

The pace pulse processing includes: limiting the bandwidth of the sampled digital signal to 2kHz by a second low-pass filtering, and extracting filtered digital signal according to the sampling rate required by the pace pulse measuring. After extracting, the sampling rate is reduced to a value more than 10 kSa/s, and the original pace detecting signal is obtained. Referring to FIG. 4, the pace pulse processing module includes a second low-pass filter 244, a second extracting unit 245, and a pace detecting original data analyzing unit 246. The second low-pass filter 244 processes the digital signal output by the shared sampling channel. The second extracting unit 245 extracts filtered digital signal according to a sampling rate required by pace pulse measuring, and outputs extracted pace pulse to the pace detecting original data analyzing unit 246.

Referring to FIG. 4, in an embodiment, ECG wave data extracting and processing includes: limiting the bandwidth of the sampled digital signal to 30Hz by a first low pass filtering to guarantee a signal-to-noise ratio of system, and extracting the filtered digital signal according to the sampling rate required by the ECG measuring. After extracting, the sampling rate is reduced to 500sa/s, and the original ECG detecting signal is obtained. In an embodiment, the ECG wave data extracting and processing module includes a first low-pass filter 247, a first extracting unit 248, and an ECG detecting original data analyzing unit 249. The first low-pass filter 247 processes the digital signal output by the shared sampling channel. The first extracting unit extracts filtered digital signal according to a sampling rate required by the ECG waveform data measuring. The first extracting unit 248 outputs ECG data to the ECG detecting original data analyzing unit 249.

The patient impedance calculating and processing module, the ECG wave data extracting and processing module, and the pace pulse processing module in the embodiment could be realized by separating hardwares, and also could be realized by software.

Experiments prove that when the front-end original data is oversampled, the noise spectrum is distributed in a wider range. Most noise could be filtered by a low-pass filtering during pace pulse processing and ECG wave data extracting and processing, thus the noise is reduced and the sampling accuracy is improved.

The analyzing module 240 determines whether electric shocking therapy is suitable for the patient according to the analyzing result of the patient impedance, ECG waveform data, and pace pulse. If the electric shocking therapy is suitable for the patient, the analyzing module 240 would control following charging and discharging.

In an embodiment, in order to improve an accuracy of the impedance measurement, a driving voltage amplitude of the AC signal is improved as could as possible. Magnifications and supply voltages of the amplifier and the delay amplifier are configured to meet the following conductions. The first conduction is a current flowing through a patient body is less than a safe current. The second conduction is the signal input into the sampling signal would not result to a saturation of the sampling channel. Superimposing a signal with a specific characteristic on the sampling signal could eliminate a conversion error of the analogy-to-digital converter, and the effect of oversampling is further improved.

From above description, the original signal is oversampled and extracted by the front-end measuring system, then the oversampled signal meets each requirement of sampling rate and accuracy, thus more than one kind of measuring parameters could use one shared sampling channel required by measured parameters The number of hardware components used in the front-end measuring system of the defibrillator could be reduced, and the number of connected nodes between hardware components could be reduced too, thus note interference could be reduced. Further the problem of coponent dispersivity caused by the large number of the hardwares could be reduced. Experiments prove that the accuracy of the result tested by the AED is improved. At the same time, volume of the AED and the cost of the AED could correspondingly be reduced.

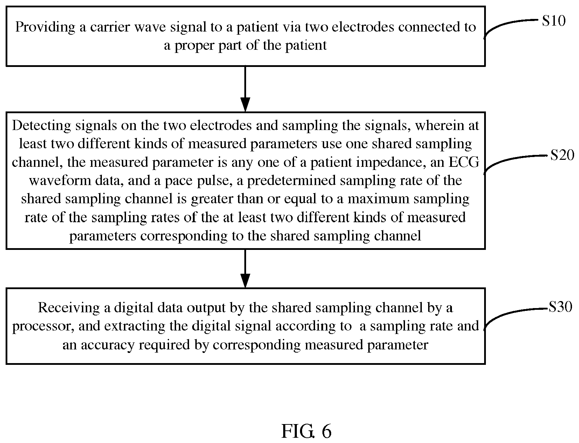

Referring to FIG. 6, a measuring method of an automatic external defibrillator includes the following steps.
Step S10, providing a carrier wave signal to a patient via two electrodes connected to a proper part of the patient.
Step S20, detecting signals on the two electrodes and sampling the signals, wherein at least two different kinds of measured parameters use one shared sampling channel, the measured parameter is any one of a patient impedance, an ECG waveform data, and a pace pulse, a predetermined sampling rate of the shared sampling channel is greater than or equal to a maximum sampling rate of the sampling rates of the at least two different kinds of measured parameters corresponding to the shared sampling channel.
Step S30, receiving a digital data output by the shared sampling channel by a processor, and extracting the digital signal according to a sampling rate and an accuracy required by corresponding measured parameter.

In an embodiment of present disclosure, after the defibrillator self tests, when the defibrillator is powered on, the defibrillator enters an AED mode, and the defibrillator automatically collects the front-end data if a testing load is not taken down. Because a testing load is connected to a front end of each electrode 21, if the defibrillator is directly connected with the human body, an erroneous analysis results may be obtained, and a treatment to the patient may be delayed. Whether the defibrillator is connected with a testing load can also be detected by power-on self-test. If the defibrillator is connected with a testing load, the impedances tested almost the same when the defibrillator is driven by two AC signals with different frequencies.

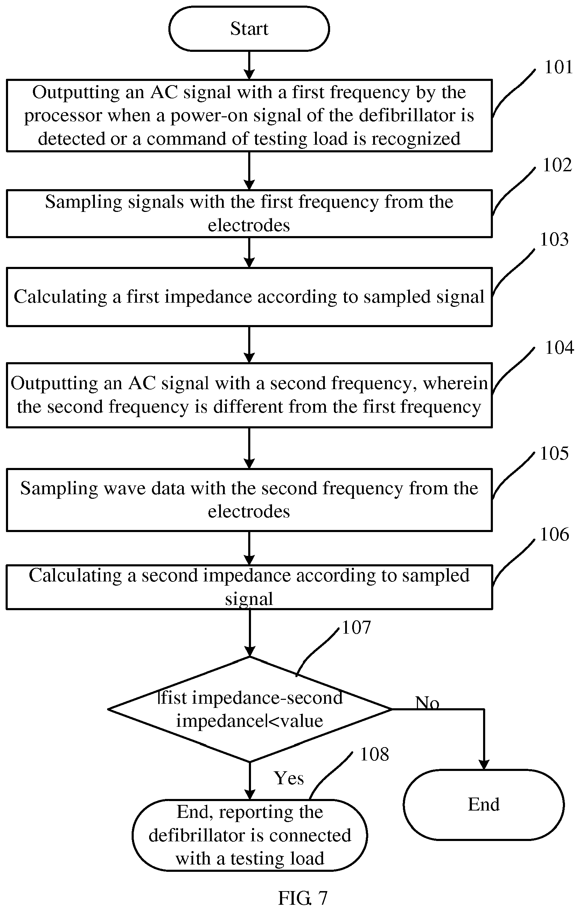

Referring to FIG. 7, recognizing a testing load includes the following steps.
Step S101, outputting an AC signal with a first frequency by the processor when a power-on signal of the defibrillator is detected or a command of testing load is recognized.
Step S102, sampling signals with the first frequency from the electrodes.
Step S103, calculating a first impedance according to sampled signal.
Step S104, outputting an AC signal with a second frequency, wherein the second frequency is different from the first frequency.
Step S105, sampling wave data with the second frequency from the electrodes.
Step S106, calculating a second impedance according to sampled signal.
Step S107, calculating an absolute value of a difference between the first impedance and the second impedance, and determining whether the absolute value is less than a predetermined value, if the absolute value is less than a predetermined value, the defibrillator is determined to be connected with a testing load.
   In detail, the predetermined value can be a very value, such as 10 Ω. If two absolute values of the difference between the first impedance and the second impedance are less than the predetermined value, the defibrillator is determined to be connected with a testing load, a step 108 is executed. Otherwise, if two absolute values of the difference between the first impedance and the second impedance are more than or equal to the predetermined value, the defibrillator is determined not to be connected with a testing load, a normal testing is carried out.
Step 108, reporting the defibrillator is connected with a testing load. The defibrillator can be used when the testing load is taken down by an operator.

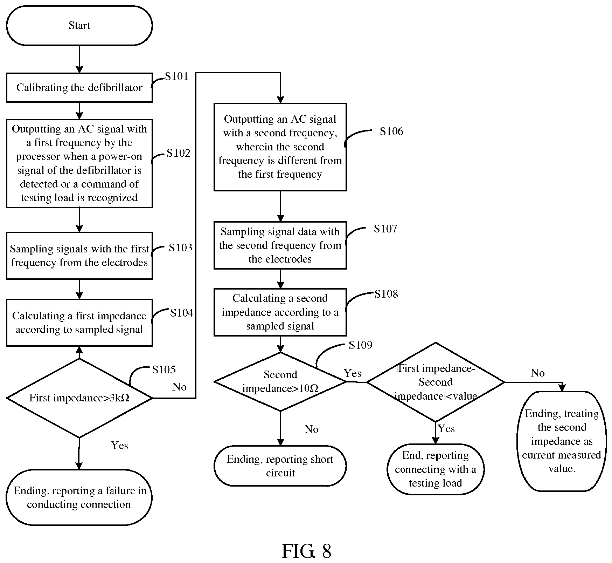

Recognizing the testing load and recognizing the patient impedance can be determined at the same time in another embodiment, and its flowchart can be referred to FIG.8.
Step S101, calibrating the defibrillator.
Step S102, outputting an AC signal with a first frequency by the processor when a power-on signal of the defibrillator is detected or a command of testing load is recognized.
Step S103, sampling signals with the first frequency from the electrodes.
Step S104, calculating a first impedance according to sampled signal.
Step S105, determining whether the first impedance is greater than 3KΩ, if the first impedance is greater than 3K Ω , ending the testing and reporting a failure in conducting connection. If the first impedance is less than 3KΩ , a step S106 is executed.
Step S106, outputting an AC signal with a second frequency, wherein the second frequency is different from the first frequency.
Step S107, sampling signal with the second frequency from the electrodes.
Step S108, calculating a second impedance according to a sampled signal.
Step S109, determining whether the second impedance is greater than 10 Ω , if the second impedance is greater than 10 Ω , ending the testing and reporting short circuit. If the second impedance is less than 10 Ω , determining whether an absolute value of a difference between the first impedance and the second impedance is less than a predetermined value, if the absolute value is less than the predetermined value, the defibrillator is determined to be connected with a testing load, the defibrillator is taken down by an operator. Otherwise, ending the testing and treating the second impedance as current measured value.

When calculating the impedance according to the sampling value, an impedance value is obtained according to a curve between a sampling voltage value and the impedance based on the sampling voltage value. However, the actual impedance is different from the impedance obtained according to the curve between the sampling voltage value and the impedance based on the sampling voltage value, because of a parameter drift of the elements of the front-end measuring system. Therefore, the front-end measuring system also includes a calibration circuit 26. As shown in FIG. 6, the calibration circuit 26 includes a switch 261, a first calibration resistance 262 and a second calibration resistance 263. Two output ports of the switch 261 are coupled to input ports of the two input protection circuits 231 respectively, and the first calibration resistance 262 or the second calibration resistance 263 is coupled between two input ports of the two input protection circuits under control of the switch. In detail, a resistance of the first calibration resistance 262 is different from a resistance of the second calibration resistance 263. The switch 261 can be a double-point switch, or can be a multiplex switch controlled by the processor.

When calibrating, the switch 261 is connected to the first calibration resistance 261, an AC signal generated by the processor is provided to the first calibration resistance 261, and a first sampling voltage value is obtained by sampling an original signal. Then the switch 261 is connected to the second calibration resistance 262, an AC signal generated by the processor is provided to the second calibration resistance 263, and a second sampling voltage value is obtained by sampling an original signal. The curve between the sampling voltage value and the impedance is updated according to the first sampling voltage value and the second sampling voltage value.

The sampling voltage value and the impedance are approximate liner relationship, when driven by the carrier wave with a certain frequency and certain voltage amplitude. A relationship between the sampling voltage and the impedance is curve because kinds inaccuracies of the system, such as dispersivities of resistance, capacitance, and integrated chip. Two points of the curve can be obtained by respectively connecting the first calibration resistance and the second calibration resistance, and a curve relationship of impedance value and the sampling value is established.

While the present disclosure has been described with reference to particular embodiments, it will be understood that the embodiments are illustrative and that the invention scope is not so limited. Alternative embodiments of the present invention will become apparent to those having ordinary skill in the art to which the present invention pertains. Accordingly, the scope of the present invention is defined by the appended claims and is supported by the foregoing description.

## Claims

1. A front-end measuring system of an automatic external defibrillator, comprising:
two electrodes (21);
a carrier wave driving circuit (22) configured to amplify a generated alternating current signal and output an amplified alternating current signal to the two electrodes (21);
a sampling circuit (23) that comprises at least one sampling channel, wherein the at least one sampling channel is a shared sampling channel, the shared sampling channel is a measuring channel shared for measuring at least two different kinds of measured parameters, the measured parameters are selected from a patient impedance, an ECG waveform data, and a pace pulse, the shared sampling channel comprises a difference amplifier (232) and an analog-to-digital converter (233), two input ports of the difference amplifier are coupled to the two electrodes (21) respectively, and an output port of the difference amplifier is coupled to the analog-to-digital converter (233), the analog-to-digital converter (233) is configured to convert an input analog signal to a digital signal by sampling the input analog signal with a predetermined sampling rate and then output the digital signal, the predetermined sampling rate of the analog-to-digital converter (233) is greater than or equal to a maximum sampling rate of the at least two different kinds of measured parameters measured with the shared sampling channel; and
a processor (24), coupled to the sampling circuit (23), which is configured to generate the alternating current signal, output the generated alternating current signal to the carrier wave driving circuit (22), receive the digital signal output by the sampling circuit (23), and extract a digital signal according to a sampling rate and an accuracy required by the measured parameters.

2. The front-end measuring system according to claim 1, wherein the shared sampling channel is shared for measuring the patient impedance,
the ECG waveform data, and the pace pulse, the predetermined sampling rate of the analog-to-digital converter (233) of the shared sampling channel is equal to a sampling rate required by patient impedance measuring, the processor (24) comprises a patient impedance calculating and processing module, an ECG wave data extracting and processing module, and a pace pulse processing module; the patient impedance calculating and processing module comprises a band pass filter and a calculating unit, the band pass filter is configured to process the digital signal output by the shared sampling channel, and the calculating unit is configured to calculate the patient impedance based on the processed digital signal;
the ECG wave data extracting and processing module comprises a first low-pass filter and a first extracting unit, the first low-pass filter is configured to process the digital signal output by the shared sampling channel, and the first extracting unit is configured to extract a filtered digital signal according to a sampling rate required by measuring the ECG waveform data and output extracted ECG data;
the pace pulse processing module comprises a second low-pass filter and a second extracting unit, the second low-pass filter is configured to process the digital signal output by the shared sampling channel, and the second extracting unit is configured to extract a filtered digital signal according to a sampling rate required by measuring the pace pulse and output an extracted pace pulse.

3. The front-end measuring system according to claim 1, wherein the sampling circuit (23) further comprises two input protection circuits (231) for absorbing high voltage energy produced by the defibrillator, and the two input protection circuits (231) are connected between two input ports of the sampling circuit (23) and the electrodes (21) respectively.

4. The front-end measuring system according to claim 3, further comprising a calibration circuit, the calibration circuit comprising a switch, a first calibration resistance and a second calibration resistance, the first calibration resistance or the second calibration resistance is coupled between two input ports of the two input protection circuits under control of the switch.

5. The front-end measuring system according to claim 1, wherein the carrier wave driving circuit (22) comprises a band pass filter (222), an amplifier (223), and a delay amplifier (224), an input port of the band pass filter (222) is coupled to an output port of the processor (24) which is configured to output the alternating current signal, an output port of the band pass filter (222) is coupled to input ports of the amplifier (223) and the delay amplifier (224) respectively, output ports of the amplifier (223) and the delay amplifier (224) are coupled to the two electrodes (21) respectively, and a phase of the altering current signal output by the delay amplifier (224) is a predetermined phase delayed compared to a phase of the altering current signal output by the amplifier (223).

6. The front-end measuring system according to claim 5, wherein the delay amplifier (224) is an inverting amplifier and a phase of the altering current signal output by the inverting amplifier is contrary to the phase of the altering current signal output by the amplifier (223).

7. The front-end measuring system according to claim 5, wherein magnifications and supply voltages of the amplifier (223) and the delay amplifier (224) are configured that a current flowing through a patient body is less than a safe current, and the signal input into the sampling channel does not result to a saturation of the sampling channel.

8. The front-end measuring system according to claim 5, wherein the processor (24) is configured to output two kinds of alternating current signals with different frequencies, and then determine whether the two electrodes (21) are well connected to a patient according to a signal received by the sampling circuit (23); or the processor (24) is configured to output two kind of alternating current signals with different frequencies by responding to a power-on signal, and then determine whether the two electrodes (21) are shot by an external conductor according to a signal received by the sampling circuit (23).

9. An automatic external defibrillator, comprising a front-end measuring system of claim 1.

10. A front-end measuring method of an automatic external defibrillator, comprising:
generating an alternating current signal via a processor; providing a carrier wave signal (S10), by amplifying the generated alternating current signal, to a patient via two electrodes connected to a proper part of the patient;
detecting and sampling signals (S20) on the two electrodes using a shared sampling channel being a measuring channel shared for measuring at least two different kinds of measured parameters, the measured parameters are any one of a patient impedance, an ECG waveform data, and a pace pulse, wherein the shared sampling channel comprises a difference amplifier and an analog-to-digital converter, two input ports of the difference amplifier are coupled to the two electrodes respectively, and an output port of the difference amplifier is coupled to the analog-to-digital converter, a predetermined sampling rate of the analog-to-digital converter is greater than or equal to a maximum sampling rate of the at least two different kinds of measured parameters measured with the shared sampling channel; and
receiving a digital signal output (S30) by the shared sampling channel via the processor, and then extracting via the processor a digital signal according to a sampling rate and an accuracy required by the measured parameters.

11. The front-end measuring method according to claim 10, wherein the shared sampling channel is shared for measuring the patient impedance, the ECG waveform data, and the pace pulse, the digital signal output by the shared sampling channel is processed via the processor by patient impedance calculating, ECG wave data extracting and processing, and pace pulse processing respectively;
the patient impedance calculating comprises band pass filtering the digital signal and calculating the patient impedance based on the filtered digital signal; the ECG wave data extracting and processing comprises a first low pass filtering the digital signal, and extracting a filtered digital signal according to a sampling rate required by measuring the ECG waveform data to obtain extracted ECG data;
the pace pulse processing comprises a second low pass filtering the digital signal, extracting a filtered digital signal according to a sampling rate required by measuring the pace pulse to obtain an extracted pace pulse.

12. The front-end measuring method according to claim 10, furthering comprising a testing load recognizing step, the testing load recognizing step comprises:
outputting an alternating current signal (101) with a first frequency;
sampling signals (102) with the first frequency from the electrodes;
calculating a first impedance (103) according to the signals sampled with the first frequency;
outputting an alternating current signal (104) with a second frequency;
sampling signals (105) with the second frequency from the electrodes;
calculating a second impedance (106) according to the signals sampled with the second frequency;
calculating an absolute value (107) of a difference between the first impedance and the second impedance, and determining whether the absolute value is less than a predetermined value, wherein if the absolute value is less than the predetermined value, the defibrillator is determined to be connected with the testing load (108).

## Patentansprüche

1. Ein Front-End-Messsystem eines automatischen externen Defibrillators mit:
zwei Elektroden (21),
einer Trägerwellen Ansteuerungsschaltung (22), die zur Verstärkung eines generierten Wechselstromsignals und zur Ausgabe eines verstärkten Wechselstromsignals an die beiden Elektroden (21) ausgelegt ist,
einer Abtastschaltung (23) mit mindestens einem Abtastkanal, wobei der mindestens eine Abtastkanal ein geteilter Abtastkanal ist, der geteilte Abtastkanal ein Messkanal ist, der zur Messung von mindestens zwei unterschiedlichen Arten gemessener Parameter geteilt wird, die gemessenen Parameter aus Patienten-Impedanz, EKG-Wellenformdaten und Schrittimpulsen gewählt werden, der geteilte Abtastkanal einen Differenzverstärker (232) und einen Analog-zu-Digital-Wandler (233) umfasst, zwei Eingänge des Differenzverstärkers an die beiden Elektroden (21) angeschlossen sind und ein Ausgang des Differenzverstärkers an den Analog-zu-Digital-Wandler (233) angeschlossen ist, der Analog-zu-Digital-Wandler (233) zur Wandlung eines analogen Signals in ein digitales Signal durch Abtasten des analogen Eingangssignals mit einer vorgegebenen Abtastrate und zur darauffolgenden Ausgabe des digitalen Signals ausgelegt ist, die vorgegebene Abtastrate des Analog-zu-Digital-Wandlers (233) größer gleich einer maximalen Abtastrate der mindestens zwei unterschiedlichen Arten über den geteilten Abtastkanal gemessener Parameter ist, und
einem an die Abtastschaltung (23) angeschlossenen Prozessor (24), der zur Generierung des Wechselstromsignals, zur Ausgabe des generierten Wechselstromsignals an die Trägerwellen Ansteuerungsschaltung (22), zum Empfang des digitalen Ausgangssignals der Abtastschaltung (23) und zur Extraktion eines digitalen Signals abhängig von einer Abtastrate und einer für die gemessenen Parameter erforderlichen Genauigkeit ausgelegt ist.

2. Das Front-End-Messsystem gemäß Anspruch 1, wobei der geteilte Abtastkanal zur Messung von Patientenimpedanz, EKG-Wellenform und Schrittimpulsen geteilt wird, die vorbestimmte Abtastrate des Analog-zu-Digital-Wandlers (233) des geteilten Abtastkanals gleich der für die Patientenimpedanzmessung erforderlichen Abtastrate ist, der Prozessor (24) ein Berechnungs- und Verarbeitungsmodul für die Patientenimpedanz umfasst, ein EKG-Wellendaten-Erfassungs- und Verarbeitungsmodul und ein Verarbeitungsmodul für Schrittimpulse,
das Berechnungs- und Verarbeitungsmodul für die Patientenimpedanz einen Bandpassfilter und eine Rechnereinheit umfasst, der Bandpassfilter so ausgelegt ist, dass er das digitale Ausgangssignal des geteilten Abtastkanals verarbeitet und die Rechnereinheit so ausgelegt ist, dass sie die Patientenimpedanz auf der Grundlage des verarbeiteten digitalen Signals berechnet,
das EKG-Wellendaten-Erfassungs- und Verarbeitungsmodul einen ersten Tiefpassfilter und eine erste Erfassungseinheit umfasst, der erste Tiefpassfilter zur Verarbeitung des vom geteilten Abtastkanal ausgegebenen digitalen Signals und die erste Erfassungseinheit zur Erfassung eines gefilterten Digitalsignals gemäß einer für die Messung der EKG-Wellenformdaten erforderlichen Abtastrate und zur Ausgabe der erfassten EKG-Daten ausgelegt ist,
das Verarbeitungsmodul für Schrittimpulse einen zweiten Tiefpassfilter und eine zweite Erfassungseinheit umfasst, der zweite Tiefpassfilter zur Verarbeitung des vom geteilten Abtastkanal ausgegebenen digitalen Signals und die zweite Erfassungseinheit zur Erfassung eines gefilterten Digitalsignals gemäß einer für die Messung der Schrittimpulse erforderlichen Abtastrate und zur Ausgabe der erfassten Schrittimpulse ausgelegt ist.

3. Das Front-End-Messsystem gemäß Anspruch 1, wobei die Abtastschaltung (23) weiterhin zwei Eingangsschutzbeschaltungen (231) zur Aufnahme der vom Defibrillator generierten Hochspannungsenergie umfasst und die beiden Eingangsschutzbeschaltungen (231) an die beiden Eingänge der Abtasteinheit (23) und die Elektroden (21) angeschlossen sind.

4. Das Front-End-Messsystem gemäß Anspruch 3, das weiterhin eine Rechnereinheit umfasst, wobei die Rechnereinheit einen Schalter enthält, einen ersten Kalibrationswiderstand und einen zweiten Kalibrationswiderstand und der erste oder der zweite Kalibrationswiderstand an die beiden Eingänge der beiden vom Schalter kontrollierten Eingangsschutzbeschaltungen angeschlossen ist.

5. Das Front-End-Messsystem gemäß Anspruch 1, wobei die Trägerwellen Ansteuerungsschaltung (22) einen Bandpassfilter (222), einen Verstärker (223) und einen Verzögerungsverstärker (224) umfasst, ein Anschluss des Bandpassfilters (222) an einen Ausgang des Prozessors (24) angeschlossen ist, der zur Ausgabe des Wechselstromsignals ausgelegt ist, ein Anschluss des Bandpassfilters (222) an die Eingänge des Verstärkers (223) bzw. des Verzögerungsverstärkers (224) angeschlossen ist, die Ausgänge des Verstärkers (223) und
des Verzögerungsverstärkers (224) an die beiden Elektroden (21) angeschlossen sind und eine Phase des vom Verzögerungsverstärker (224) ausgegebenen Wechselstromsignals eine im Vergleich zu einer Phase des vom Verstärker (223) ausgegebenen Wechselstromsignal vorbestimmte verzögerte Phase ist.

6. Das Front-End-Messsystem gemäß Anspruch 5, wobei der Verzögerungsverstärker (224) ein invertierender Verstärker ist und sich eine Phase des vom invertierenden Verstärker ausgegebenen Wechselstromsignals zur Phase des vom Verstärker (223) ausgegebenen Wechselstromsignals entgegengesetzt verhält.

7. Das Front-End-Messsystem gemäß Anspruch 5, wobei Erhöhung und Spannungsversorgung von Verstärker (223) und Verzögerungsverstärker (224) so ausgelegt sind, dass ein durch den Körper eines Patienten fließender Strom unter einem sicheren Strom liegt und das Eingangssignal für den Abtastkanal nicht zu einer Sättigung des Abtastkanals führt.

8. Das Front-End-Messsystem gemäß Anspruch 5, wobei der Prozessor (24) so ausgelegt ist, dass er zwei Arten von Wechselstromsignalen mit unterschiedlichen Frequenzen ausgibt und danach anhand des von der Abtastschaltung (23) empfangenen Signals bestimmt, ob die beiden Elektroden (21) tatsächlich an den Patienten angeschlossen sind oder
der Prozessor (24) so ausgelegt ist, dass er als Reaktion auf ein Spannung EIN-Signal zwei Arten von Wechselstromsignalen mit unterschiedlichen Frequenzen ausgibt und danach anhand des von der Abtastschaltung (23) empfangenen Signals bestimmt, ob die beiden Elektroden (21) von einem Außenleiter beschossen werden.

9. Ein automatischer externer Defibrillator mit einem Front-End-Messsystem gemäß Anspruch 1.

10. Ein Front-End-Messverfahren eines automatischen externen Defibrillators mit:
der Generation eines Wechselstromsignals über einen Prozessor,
der Bereitstellung eines Trägerwellensignals (S10) durch Verstärkung des generierten Wechselstromsignals an einen Patienten über zwei an ein Körperteil des Patienten angeschlossene Elektroden,
Erfassungs- und Abtastsignalen (S20) an den beiden Elektroden, die einen geteilten Abtastkanal verwenden, der zur Messung von mindestens zwei unterschiedlichen Arten gemessener Parameter geteilt wird, die gemessenen Parameter aus Patienten-Impedanz, EKG-Wellenformdaten oder Schrittimpulsen gewählt werden, der geteilte Abtastkanal einen Differenzverstärker und einen Analog-zu-Digital-Wandler umfasst, zwei Eingänge des Differenzverstärkers an die beiden Elektroden angeschlossen sind und ein Ausgang des Differenzverstärkers an den Analog-zu-Digital-Wandler angeschlossen ist, eine vorgegebene Abtastrate des Analog-zu-Digital-Wandlers größer gleich einer maximalen Abtastrate der mindestens zwei unterschiedlichen Arten über den geteilten Abtastkanal gemessener Parameter ist, und
dem Empfang eines digitalen, vom geteilten Abtastkanal über den Prozessor ausgegebenen Signals (S30) und anschließender Extraktion des digitalen Signals gemäß einer von den gemessenen Parametern erforderlichen Abtastrate und Genauigkeit.

11. Das Front-End-Messverfahren gemäß Anspruch 10, wobei der geteilte Abtastkanal zur Messung von Patientenimpedanz, EKG-Wellenformdaten und Schrittimpulsen geteilt wird, das vom geteilten Abtastkanal ausgegebene digitale Signal über den Prozessor durch Berechnung der Patientenimpedanz, Extraktion und Verarbeitung von EKG-Wellendaten und Verarbeitung von Schrittimpulsen verarbeitet wird,
die Berechnung der Patientenimpedanz eine Bandpassfilterung des digitalen Signals beinhaltet und die Berechnung der Patientenimpedanz auf dem gefilterten digitalen Signal beruht,
die Extraktion und Verarbeitung der EKG-Wellendaten eine erste Tiefpassfilterung des digitalen Signals und die Extraktion eines gefilterten digitalen Signals mit der für die Messung von EKG-Wellenformdaten und zur Erlangung der extrahierten EKG-Daten erforderlichen Abtastrate umfasst,
die Verarbeitung der Schrittimpulse eine zweite Tiefpassfilterung des digitalen Signals umfasst,
sowie die Extraktion eines gefilterten digitalen Signals mit einer für die Messung der Schrittimpulse zur Erlangung eines extrahierten Schrittimpulses erforderlichen Abtastrate.

12. Das Front-End-Messverfahren gemäß Anspruch 10, das weiterhin einen Schritt zur Erkennung einer Prüflast umfasst, wobei der Schritt zur Erkennung der Prüflast Folgendes umfasst:
die Ausgabe eines Wechselstromsignals (101) mit einer ersten Frequenz,
das Abtasten von Signalen (102) mit der ersten Frequenz von den Elektroden,
die Berechnung einer ersten Impedanz (103) anhand der mit der ersten Frequenz abgetasteten Signale,
die Ausgabe eines Wechselstromsignals (104) mit einer zweiten Frequenz,
das Abtasten von Signalen (105) mit der zweiten Frequenz von den Elektroden,
die Berechnung einer zweiten Impedanz (106) anhand der mit der zweiten Frequenz abgetasteten Signale,
die Berechnung eines Absolutwerts (107) der Differenz zwischen der ersten und der zweiten Impedanz und die Bestimmung, ob der Absolutwert unter einem festgelegten Wert liegt, wobei, falls der Absolutwert unter dem festgelegten Wert liegt, der Defibrillator als mit der Prüflast (108) verbunden angesehen wird.

## Revendications

1. Système de mesure frontal d'un défibrillateur externe automatique, comprenant :
deux électrodes (21) ;
un circuit d'attaque à onde porteuse (22) configuré pour amplifier un signal de courant alternatif généré et fournir en sortie un signal de courant alternatif amplifié aux deux électrodes (21) ;
un circuit d'échantillonnage (23) qui comprend au moins un canal d'échantillonnage, où le au moins un canal d'échantillonnage est un canal d'échantillonnage partagé, le canal d'échantillonnage partagé est un canal de mesure partagé pour mesurer au moins deux sortes différentes de paramètres mesurés, les paramètres mesurés sont sélectionnés parmi une impédance de patient, une donnée de forme d'onde d'ECG, et un pouls électro-entraîné, le canal d'échantillonnage partagé comprend un amplificateur de différence (232) et un convertisseur analogique-numérique (233), deux bornes d'entrée de l'amplificateur de différence sont couplées aux deux électrodes (21) respectivement, et une borne de sortie de l'amplificateur de différence est couplée au convertisseur analogique-numérique (233), le convertisseur analogique-numérique (233) est configuré pour convertir un signal analogique d'entrée en un signal numérique en échantillonnant le signal analogique d'entrée à une fréquence d'échantillonnage prédéterminée et ensuite fournir en sortie le signal numérique, la fréquence d'échantillonnage prédéterminée du convertisseur analogique-numérique (233) est supérieure ou égale à une fréquence d'échantillonnage maximale des au moins deux sortes différentes de paramètres mesurés qui sont mesurés au moyen du canal d'échantillonnage partagé ; et
un processeur (24), couplé au circuit d'échantillonnage (23), lequel est configuré pour générer le signal de courant alternatif, fournir en sortie le signal de courant alternatif généré au circuit d'attaque à onde porteuse (22), recevoir le signal numérique fourni en sortie par le circuit d'échantillonnage (23), et extraire un signal numérique selon une fréquence d'échantillonnage et une précision requises par les paramètres mesurés.

2. Système de mesure frontal selon la revendication 1, dans lequel le canal d'échantillonnage partagé est partagé pour mesurer l'impédance de patient, la donnée de forme d'onde d'ECG, et le pouls électro-entraîné, la fréquence d'échantillonnage prédéterminée du convertisseur analogique-numérique (233) du canal d'échantillonnage partagé est égale à une fréquence d'échantillonnage requise par la mesure d'impédance de patient, le processeur (24) comprend un module de calcul et de traitement d'impédance de patient, un module d'extraction et de traitement de donnée d'onde d'ECG, et un module de traitement de pouls électro-entraîné ;
le module de calcul et de traitement d'impédance de patient comprend un filtre passe-bande et une unité de calcul, le filtre passe-bande est configuré pour traiter le signal numérique fourni en sortie par le canal d'échantillonnage partagé, et l'unité de calcul est configurée pour calculer l'impédance de patient sur la base du signal numérique traité ;
le module d'extraction et de traitement de donnée d'onde d'ECG comprend un premier filtre passe-bas et une première unité d'extraction, le premier filtre passe-bas est configuré pour traiter le signal numérique fourni en sortie par le canal d'échantillonnage partagé, et la première unité d'extraction est configurée pour extraire un signal numérique filtré selon une fréquence d'échantillonnage requise par la mesure de la donnée de forme d'onde d'ECG et fournir en sortie des données d'ECG extraites ;
le module de traitement de pouls électro-entraîné comprend un second filtre passe-bas et une seconde unité d'extraction, le second filtre passe-bas est configuré pour traiter le signal numérique fourni en sortie par le canal d'échantillonnage partagé, et la seconde unité d'extraction est configurée pour extraire un signal numérique filtré selon une fréquence d'échantillonnage requise par la mesure du pouls électro-entraîné et fournir en sortie un pouls électro-entraîné extrait.

3. Système de mesure frontal selon la revendication 1, dans lequel le circuit d'échantillonnage (23) comprend en outre deux circuits de protection d'entrée (231) destinés à absorber une énergie de tension élevée produite par le défibrillateur, et les deux circuits de protection d'entrée (231) sont reliés entre deux bornes d'entrée du circuit d'échantillonnage (23) et les électrodes (21) respectivement.

4. Système de mesure frontal selon la revendication 3, comprenant en outre un circuit d'étalonnage, le circuit d'étalonnage comprenant un commutateur, une première résistance d'étalonnage et une seconde résistance d'étalonnage, le première résistance d'étalonnage ou la seconde résistance d'étalonnage est couplée entre deux bornes d'entrée des deux circuits de protection d'entrée sous le contrôle du commutateur.

5. Système de mesure frontal selon la revendication 1, dans lequel le circuit d'attaque à onde porteuse (22) comprend un filtre passe-bande (222), un amplificateur (223), et un amplificateur de retard (224), une borne d'entrée du filtre passe-bande (222) est couplée à une borne de sortie du processeur (24) lequel est configuré pour fournir en sortie le signal de courant alternatif, une borne de sortie du filtre passe-bande (222) est couplée à des bornes d'entrée de l'amplificateur (223) et de l'amplificateur de retard (224) respectivement, des bornes de sortie de l'amplificateur (223) et de l'amplificateur de retard (224) sont couplées aux deux électrodes (21) respectivement, et une phase du signal de courant alternatif fourni en sortie par l'amplificateur de retard (224) est une phase prédéterminée en retard par rapport à une phase du signal de courant alternatif fourni en sortie par l'amplificateur (223).

6. Système de mesure frontal selon la revendication 5, dans lequel l'amplificateur de retard (224) est un amplificateur inverseur et une phase du signal de courant alternatif fourni en sortie par l'amplificateur inverseur est contraire à la phase du signal de courant alternatif fourni en sortie par l'amplificateur (223).

7. Système de mesure frontal selon la revendication 5, dans lequel des amplifications et des tensions d'alimentation de l'amplificateur (223) et de l'amplificateur de retard (224) sont configurées pour qu'un courant circulant à travers un corps de patient soit inférieur à un courant sûr, et que le signal appliqué en entrée au canal d'échantillonnage ne provoque pas de saturation du canal d'échantillonnage.

8. Système de mesure frontal selon la revendication 5, dans lequel le processeur (24) est configuré pour fournir en sortie deux sortes de signaux de courant alternatif avec des fréquences différentes, et ensuite déterminer si les deux électrodes (21) sont correctement reliées à un patient ou non selon un signal reçu par le circuit d'échantillonnage (23) ; ou
le processeur (24) est configuré pour fournir en sortie deux sortes de signaux de courant alternatif avec des fréquences différentes en réponse à un signal de mise sous tension, et ensuite déterminer si les deux électrodes (21) sont reliées par un conducteur externe selon un signal reçu par le circuit d'échantillonnage (23).

9. Défibrillateur externe automatique, comprenant un système de mesure frontal selon la revendication 1.

10. Procédé de mesure frontal d'un défibrillateur externe automatique, comprenant les étapes consistant à :
générer un signal de courant alternatif via un processeur ;
fournir un signal d'onde porteuse (S 10), en amplifiant le signal de courant alternatif généré, à un patient via deux électrodes reliées à une partie adéquate du patient ;
détecter et échantillonner des signaux (S20) sur les deux électrodes en utilisant un canal d'échantillonnage partagé qui est un canal de mesure partagé pour mesurer au moins deux sortes différentes de paramètres mesurés, les paramètres mesurés sont l'un quelconque d'une impédance de patient, d'une donnée de forme d'onde d'ECG, et d'un pouls électro-entraîné, où le canal d'échantillonnage partagé comprend un amplificateur de différence et un convertisseur analogique-numérique, deux bornes d'entrée de l'amplificateur de différence sont couplées aux deux électrodes respectivement, et une borne de sortie de l'amplificateur de différence est couplée au convertisseur analogique-numérique, une fréquence d'échantillonnage prédéterminée du convertisseur analogique-numérique est supérieure ou égale à une fréquence d'échantillonnage maximale des au moins deux sortes différentes de paramètres mesurés qui sont mesurés au moyen du canal d'échantillonnage partagé ; et
recevoir un signal numérique fourni en sortie (S30) par le canal d'échantillonnage partagé via le processeur (24), et ensuite extraire via le processeur un signal numérique selon une fréquence d'échantillonnage et une précision requises par les paramètres mesurés.

11. Procédé de mesure frontal selon la revendication 10, dans lequel le canal d'échantillonnage partagé est partagé pour mesurer l'impédance de patient, la donnée de forme d'onde d'ECG, et le pouls électro-entraîné, le signal numérique fourni en sortie par le canal d'échantillonnage partagé est traité via le processeur par calcul d'impédance de patient, extraction et traitement de donnée d'onde d'ECG, et traitement de pouls électro-entraîné, respectivement ;
le calcul d'impédance de patient comprend un filtrage passe-bande du signal numérique et un calcul d'impédance de patient sur la base du signal numérique filtré ;
l'extraction et traitement de donnée d'onde d'ECG comprend un premier filtrage passe-bas du signal numérique, et le fait d'extraire un signal numérique filtré selon une fréquence d'échantillonnage requise par la mesure de la donnée de forme d'onde d'ECG afin d'obtenir des données d'ECG extraites ;
le traitement de pouls électro-entraîné comprend un second filtrage passe-bas du signal numérique, et le fait d'extraire un signal numérique filtré selon une fréquence d'échantillonnage requise par la mesure du pouls électro-entraîné afin d'obtenir un pouls électro-entraîné extrait.

12. Procédé de mesure frontal selon la revendication 10, comprenant en outre une étape de reconnaissance de charge d'essai, l'étape de reconnaissance de charge d'essai comprend :
le fait de fournir en sortie un signal de courant alternatif (101) avec une première fréquence ;
le fait d'échantillonner des signaux (102) avec la première fréquence à partir des électrodes ;
le fait de calculer une première impédance (103) selon les signaux échantillonnés avec la première fréquence ;
le fait de fournir en sortie un signal de courant alternatif (104) avec une seconde fréquence ;
le fait d'échantillonner des signaux (105) avec la seconde fréquence à partir des électrodes ;
le fait de calculer une seconde impédance (106) selon les signaux échantillonnés avec la seconde fréquence ;
le fait de calculer une valeur absolue (107) d'une différence entre la première impédance et la seconde impédance, et le fait de déterminer si la valeur absolue est inférieure ou non à une valeur prédéterminée, où si la valeur absolue est inférieure à la valeur prédéterminée, il est identifié que le défibrillateur est relié à la charge d'essai (108).
